# EUROPEAN PATENT APPLICATION

(11) **EP 4 394 036 A1**
(43) Date of publication of application: **03.07.2024**
(21) Application number: 21964424.2
(22) Date of filing: 19.11.2021
(51) Int. Cl.: C12N 11/087, C12N 9/02, C12N 9/04, C12N 9/06, C12N 9/10, C08F 8/00, C08F 20/10

(54) **ENZYME IMMOBILIZATION CARRIER AND PREPARATION METHOD THEREFOR, AND IMMOBILIZED ENZYME AND PREPARATION METHOD THEREFOR**

(71) Applicant: Liaoning Asymchem Laboratories Co., Ltd., Fuxin, Liaoning 123129 (CN)
(72) Inventor: HONG, Hao, Morrisville, North Carolina 27560 (US); JAMES, Gage, Morrisville, North Carolina 27560 (US); XIAO, Yi, Tianjin 300457 (CN); ZHANG, Na, Tianjin 300457 (CN); VYASA, Williams, Morrisville, North Carolina 27560 (US); ZHAO, Jiadong, Tianjin 300457 (CN); PAN, Long, Tianjin 300457 (CN); MA, Liteng, Tianjin 300457 (CN); CUI, Yuxia, Tianjin 300457 (CN)
(74) Representative: Berggren Oy
(86) International application number: PCT/CN2021/131878
(87) International publication number: WO 2023/087270

(57) **Abstract**

Provided are an enzyme immobilization carrier and a preparation method therefor, and an immobilized enzyme and a preparation method therefor. The enzyme immobilization carrier includes a resin ball matrix, an -N(CH₂COOH)₂ group linked on the resin ball matrix by means of a chemical bond, and a metal ion adsorbed on the -N(CH₂COOH)₂ group in a chelating manner through coordination. The resin ball matrix is an amino-type methacrylic resin and/or an epoxy-type methacrylic resin, and the resin ball matrix has a particle size of 200-700 µm. Therefore, the problems in the related art that an enzyme immobilization carrier is relatively poor in enzyme compatibility and poor in immobilization effect, and an immobilized enzyme is relatively poor in activity, and relatively poor in stability during repeated use are solved.

## Description

### Technical Field

The present disclosure relates to the field of biological catalysis, and specifically, to an enzyme immobilization carrier and a preparation method therefor, and an immobilized enzyme and a preparation method therefor.

### Background

Biological catalysis has become an important part of green synthetic drugs, and is one of the most promising and applied technologies for catalytic synthesis of drug structure units and intermediates, especially for chiral synthesis, which provides a unique and irreplaceable method. Enzymes are increasingly used as catalysts in industrial processes. Since conditions of using the enzymes are mild, and the enzymes are easy to vary, requirements of the enzymes for environments are very strict, and the enzymes are difficult to recycle, such that the application of the enzymes in industry is greatly limited. The development and application of an immobilized enzyme technology may effectively solve these problems, such that an immobilized enzyme, which is often called "a biological catalyst", is widely used in industrial organic synthesis and bioconversion.

An enzyme immobilization method may be classified into a physical method and a chemical method. The physical method mainly includes an adsorption method and an embedding method; and the chemical method mainly includes a binding method and a cross-linking method. The adsorption method is also called a non-covalent method, which is an immobilization method in which an enzyme is bound to an adsorbent medium mainly through interactions of several non-covalent bonds, such as a hydrogen bond, Van Der Waals force, hydrophobic interaction, and an ionic bond. Commonly used inorganic adsorption materials include silica gel, alumina, porous glass, diatomaceous earth, etc.; commonly used organic adsorption materials mainly include natural alginate, chitin, chitosan, cellulose, and starch; and synthetic organic materials include polyurethane, macroporous resins, etc. The embedding method is to embed free enzymes into the pores of a medium such as specific gel, to achieve immobilization, for example, performing embedding by using gel media such as polyacrylamide and calcium alginate. The embedding method is simpler, an embedded enzyme protein structure may remain basically unchanged, and the loss of enzyme activity is relatively small, but there are certain disadvantages, such as the leakage of enzyme components and enzyme inactivation. The binding method, also known as covalent immobilization, is based on a reaction between an effective functional group of a carrier material and an enzyme-related functional group (for example, enzyme-protein side-chain groups such as amino, carboxyl, sulfhydryl, and hydroxyl), to achieve mutual binding. The covalent immobilization makes the binding between the enzyme and the carrier stronger, and a strong chemical bond generated between the enzyme and the carrier may significantly reduce the loss of the enzyme, such that the reuse rate of the enzyme is improved. However, the method is strict in condition, intense in reaction, and large in enzyme activity loss (typical residual enzyme activity is around 30%). The cross-linking method refers to a method of performing a cross-linking reaction between enzyme molecules, and between the enzyme molecule and the carrier by means of a bifunctional or multifunctional reagent (a cross-linking reagent), to form a covalent bond for enzyme immobilization. Commonly used cross-linking reagents include glutaraldehyde, hexamethylene diamine, maleic anhydride, double azobenzene, etc. More research has been done on Cross-Linked Enzyme Aggregates (CLEA), which are formed by cross-linking the enzymes using a cross-linking agent, such as CLEA immobilization of a phenylalanine ammonia lyase, and CLEA immobilization of a sucrose phosphorylase. To sum up, the traditional immobilization methods have many disadvantages, for example, adsorption-immobilized enzymes are easily lost, substrates and products of the enzymes after embedding immobilization are uneasy to disperse, a covalent bonding method easily leads to enzyme inactivation, and the mechanical properties of cross-linked enzymes are relatively poor.

However, most of commercial affinity resins are protein purification resins, of which matrices are usually cellulose, cross-linked dextran, agarose, polyacrylamide, porous glass beads, etc., and agarose gels are the most widely used. For example, the small particle size of a metal-chelating affinity resin makes it difficult to recycle for large-scale application, and particles are easily broken under high shear, such that the resin is not easy to be applied in a traditional stirred tank reaction mode. Meanwhile, the matrix of the resin is very hydrophilic, and has a high requirement for a storage condition, such that the resin needs to be stored in an alcohol solution, and avoids drying as much as possible, causing the resin to be unsuitable for use as an immobilized enzyme. In addition, the industrial immobilized enzyme application of the resin is greatly affected due to high cost.

Therefore, it is necessary to provide a new enzyme immobilization carrier with better compatibility to enzymes and better immobilization effects. In addition, in the chemical reaction application of an immobilized enzyme, the activity of the enzyme is reserved to a great extent, and the enzyme has higher stability during reuse.

### Summary

The present disclosure is mainly intended to provide an enzyme immobilization carrier and a preparation method therefor, and an immobilized enzyme and a preparation method therefor, so as to solve the problems in the related art that an enzyme immobilization carrier is relatively poor in enzyme compatibility and poor in immobilization effect, and an immobilized enzyme is relatively poor in activity, and relatively poor in stability during repeated use.

In order to implement the above objectives, an aspect of the present disclosure provides an enzyme immobilization carrier. The enzyme immobilization carrier includes a resin ball matrix, an -N(CH₂COOH)₂ group linked on the resin ball matrix by means of a chemical bond, and a metal ion adsorbed on the -N(CH₂COOH)₂ group in a chelating manner through coordination. The resin ball matrix is an amino-type methacrylic resin or an epoxy-type methacrylic resin, and the resin ball matrix has a particle size of 200-700 µm.

Further, in the amino-type methacrylic resin, the amino-type methacrylic resin has an amino functional group with a C2 or C6 length carbon chain arm, and the content of the amino is 30-80 µmol/g. Preferably, the amino-type methacrylic resin is one or more of Seplite^{®}LX-1000HA, Seplite ^{®}LX-1000EPN, Seplite^{®}LX-EPHA, Seplite^{®}LX-1000EA, Lifetech ^{™}ECR8309, Lifetech^{™}ECR8409, ESR-1, ESR-3, or ESQ-1. More preferably, the amino-type methacrylic resin is one or more of Seplite^{®}LX-1000HA, Seplite^{®}LX-1000EPN, Seplite^{®}LX-EPHA, or Lifetech^{™}ECR8309. Preferably, the epoxy-type methacrylic resin has an epoxide equivalent of 2-5 µmol/g. More preferably, the epoxy-type methacrylic resin is one or more of Seplite^{®}LX-1000EP, Seplite^{®}LX-103B, EP200, Seplite ^{®} LX-107B, Seplite ^{®} LX-1000SW, Seplite ^{®} LX-1000SD, Seplite ^{®} LX-109s, Seplite ^{®} LX-1000HFA, Lifetech^{™}ECR8285, Lifetech^{™}ECR8204, Lifetech^{™}ECR8209, ES-1, ES103, ES-101, ReliZyme^{™}HFA403, or ReliZyme^{™}EC-HFA. More preferably, the epoxy-type methacrylic resin is one or more of Seplite^{®}LX-1000EP, Seplite^{®}LX-109s, Seplite^{®}LX-1000HFA, or ReliZyme^{™}EC-HFA. Preferably, the metal ion is a nickel ion, an iron ion, a copper ion, or a cobalt ion.

In order to implement the above objectives, an aspect of the present disclosure provides an immobilized enzyme. The immobilized enzyme includes the enzyme immobilization carrier, and an enzyme immobilized thereon.

Further, the enzyme is selected from any one or more of a transaminase, a ketoreductase, an alcohol dehydrogenase, a formate dehydrogenase (FDH), a glucose dehydrogenase, a monooxygenase, an ene-reductase, an imine reductase, and an amino acid dehydrogenase. The transaminase is a transaminase derived from *Chromobacterium violaceum DSM30191,* or a transaminase derived from *Arthrobacter citreus,* or a transaminase derived from *Actinobacteria,* or a transaminase derived from *Sciscionella sp. SE31.* The ketoreductase is a carbonyl reductase derived from *Acetobacter sp.CCTCC M209061,* or a ketoreductase derived from *Sporobolomyces salmonicolor.* The alcohol dehydrogenase is derived from *Thermoanaerobium brockii.* The FDH is an FDH derived from *Candida boidinii.* The glucose dehydrogenase is a glucose dehydrogenase derived from *Lysinibacillus sphaericus G10.* The monooxygenase is a cyclohexanone monooxygenase derived from *Rhodococcus sp.Phi1*, or a cyclohexanone monooxygenase derived from *Rhodococcus ruber-SD1*, or a cyclohexanone monooxygenase derived from *Brachymonas petroleovorans.* The ene-reductase is an ene-reductase derived from *Saccharomyces cerevisiae,* or an ene-reductase derived from *Chryseobacterium sp.CA49.* The imine reductase is an imine reductase derived from *Streptomyces sp.,* or an imine reductase derived from *Bacillus cereus.* The amino acid dehydrogenase is a leucine dehydrogenase derived from *Bacillus cereus,* or a phenylalanine dehydrogenase derived from *Bacillus sphaericus;* or an amino acid dehydrogenase derived from *Thermoactinomyces intermedius ATCC33205,* or an amino acid dehydrogenase derived from *Thermosyntropha lipolytica.*

Another aspect of the present disclosure provides a method for preparing the enzyme immobilization carrier. The method includes the following steps: providing a resin ball matrix; linking an -N(CH₂COOH)₂ group onto the resin ball matrix by means of a chemical bond; absorbing a metal ion onto the -N(CH₂COOH)₂ group in a chelating manner through coordination, so as to obtain the enzyme immobilization carrier. The resin ball matrix is an amino-type methacrylic resin or an epoxy-type methacrylic resin, and the resin ball matrix has a particle size of 200-700 µm.

Further, when the resin ball matrix is the amino-type methacrylic resin, the amino-type methacrylic resin is mixed with sodium chloroacetate for nucleophilic substitution, so as to link the -N(CH₂COOH)₂ group onto the amino-type methacrylic resin.

Further, after an aqueous solution of the amino-type methacrylic resin and the sodium chloroacetate is stirred for 30-60 min at 20-25 °C, the reaction system is regulated to a pH of 9-10 with 1-2 mol/L of an aqueous alkaline solution, and then heated up to 70-80 °C for reaction for 20-30 h at an N₂ atmosphere, so as to link the -N(CH₂COOH)₂ group onto the amino-type methacrylic resin. Preferably, the aqueous alkali solution is a sodium carbonate solution, a sodium hydroxide solution, or a lithium hydroxide solution.

Further, when the resin ball matrix is the epoxy-type methacrylic resin, the epoxy-type methacrylic resin is mixed with Iminodiacetic acid disodium for an addition reaction, so as to link the -N(CH₂COOH)₂ group onto the epoxy-type methacrylic resin.

Further, after an aqueous solution of the epoxy-type methacrylic resin and the Iminodiacetic acid disodium is stirred for 30-60 min at 20-25 °C, the reaction system is heated up to 60-70 °C for reaction for 18-24 h at an N₂ atmosphere, so as to link the -N(CH₂COOH)₂ group onto the epoxy-type methacrylic resin.

Further, after the step of linking the -N(CH₂COOH)₂ group onto the resin ball matrix by means of the chemical bond, a metal salt solution is added to the reaction system for a complex reaction, so as to absorb the metal ion on the -N(CH₂COOH)₂ group in the chelating manner through coordination, thereby obtaining the enzyme immobilization carrier. Preferably, during the complex reaction, a reaction temperature is 20-30 °C and reaction time is 1-4h. Preferably, the metal salt solution is an aqueous solution of nickel chloride, an aqueous solution of copper sulfate, an aqueous solution of ferrous chloride, or an aqueous solution of cobalt chloride. Preferably, in the metal salt solution, a mass ratio of the metal ion to the resin ball matrix is (0.05-0.1):1.

Further, a mass ratio of the sodium chloroacetate to the amino-type methacrylic resin ball is (5-10):1.

Further, a mass ratio of the Iminodiacetic acid disodium to the epoxy-type methacrylic resin ball is (5-10):1.

Another aspect of the present disclosure provides a method for preparing the immobilized enzyme. The method includes the following step: performing an immobilization reaction on the enzyme immobilization carrier, and an enzyme, so as to obtain the immobilized enzyme.

Further, the method for preparing the immobilized enzyme includes the following steps: dispersing the enzyme immobilization carrier into a first solvent to form dispersion liquid, where the first solvent is a mixed solution of a phosphate buffer solution, a sodium chloride aqueous solution, and an imidazole buffer solution; and reacting the dispersion liquid with an enzyme solution containing the enzyme, to cause the enzyme and the enzyme immobilization carrier to be subjected to an immobilization reaction, so as to obtain the immobilized enzyme.

Further, in the first solvent, the phosphate buffer solution has the concentration of 0.1-0.2 mol/L; the sodium chloride aqueous solution has the concentration of 0.5-1 mol/L; and the imidazole buffer solution has the concentration of 0.05-0.1 mol/L.

Further, during the reaction process of the dispersion liquid and the enzyme solution, a reaction temperature is 20-25 °C, and a reaction time is 16-24h. Preferably, per gram of the enzyme immobilization carrier is reacted with 4-8 mL of the enzyme solution, and the enzyme solution has a protein content of 20-25 mg/mL.

In the carrier of the present disclosure, the amino-type methacrylic resin or the epoxy-type methacrylic resin is used as the resin ball matrix; the -N(CH₂COOH)₂ group is linked onto the resin ball matrix by means of the chemical bond; and the metal ion adsorbed on the -N(CH₂COOH)₂ group through coordination is also linked to the resin ball matrix. By using the carrier, first, the particle size (200-700 µm) of the resin ball matrix is larger than that (20-80 µm) of traditional affinity resin, such that better repeatable recyclability is achieved. In addition, the resin ball matrix has higher mechanical strength than general affinity resins, so as to prevent the resin ball matrix from being broken under high shear, such that under a traditional stirred tank reaction mode, the immobilized enzyme is longer in service life, and better in repeatable recyclability. Second, compared with a general covalent binding carrier, for a formed metal-chelating affinity structure containing a nitrogen diacetate group and the metal ion, the carrier only binds to a His tag at the end of the protein, such that the carrier is better in enzyme compatibility, the activity of the enzyme can be reserved to a great extent during immobilization, and the stability of the enzyme during reuse is higher. In addition, the resin of the above type is lower in cost. Based on this, the carrier of the present disclosure is better in enzyme compatibility and better in immobilization effect, and the activity of the enzyme can be reserved to a great extent during immobilization. Therefore, the immobilized enzyme obtained through immobilization is better in activity and higher in stability during reuse, and is more suitable for application in industrialized immobilized enzymes. Based on this, the present disclosure effectively solves the problems in the related art that an enzyme immobilization carrier is relatively poor in enzyme compatibility and poor in immobilization effect, and an immobilized enzyme is relatively poor in activity, and relatively poor in stability during repeated use.

### Detailed Description of the Embodiments

It is to be noted that the embodiments in the present application and the features in the embodiments may be combined with one another without conflict. The present disclosure will be described below in detail with reference to the embodiments.

As described in the Background, the problems that an enzyme immobilization carrier is relatively poor in enzyme compatibility and poor in immobilization effect, and an immobilized enzyme is relatively poor in activity, and relatively poor in stability during repeated use exist in the related art.

In order to solve the above problems, the present disclosure provides an enzyme immobilization carrier. The enzyme immobilization carrier includes a resin ball matrix, an -N(CH₂COOH)₂ group linked on the resin ball matrix by means of a chemical bond, and a metal ion adsorbed on the -N(CH₂COOH)₂ group in a chelating manner through coordination. The resin ball matrix is an amino-type methacrylic resin or an epoxy-type methacrylic resin, and the resin ball matrix has a particle size of 200-700 µm.

In the carrier of the present disclosure, the amino-type methacrylic resin or the epoxy-type methacrylic resin is used as the resin ball matrix; the -N(CH₂COOH)₂ group is linked onto the resin ball matrix by means of the chemical bond; and the metal ion adsorbed on the -N(CH₂COOH)₂ group through coordination is also linked to the resin ball matrix. By using the carrier, in the subsequent reaction process of the immobilized enzyme, a His tag at the end of the enzyme may be adsorbed on the metal ion through coordination. Specifically, the N(CH₂COOH)₂ group and the metal ion form a coordinate bond. However, the -N(CH₂COOH)₂ group does not completely occupy the coordination site of the metal ion, the enzyme with the His tag continues to occupy the remaining coordination sites, which are not occupied, on the metal ion, and the immobilized enzyme is formed with the carrier through coordination. Compared with covalent bonding, a chemical reaction between an enzyme body and the carrier leads to destruction of the enzyme body, and the rigidity of the immobilized enzyme becomes stronger, resulting in lower enzyme activity. However, unlike the above, in the present disclosure, by using the form of coordination binding of the His tag at the end of the enzyme and the metal ion in the carrier, there is no reaction contact between the carrier and the enzyme body, such that the loss of enzyme activity is small.

In particular, the resin ball matrix of the present disclosure is an amino-type methacrylic resin and/or an epoxy-type methacrylic resin. Based on this, first, the particle size of the resin ball matrix is larger than that of traditional affinity resin, such that better repeatable recyclability is achieved. In addition, the resin ball matrix has higher mechanical strength, so as to prevent the resin ball matrix from being broken under high shear, such that under a traditional stirred tank reaction mode, the immobilized enzyme is longer in service life, and better in repeatable recyclability. Second, the formed carrier is better in enzyme compatibility, the activity of the enzyme can be reserved to a great extent during immobilization, and the stability of the enzyme during reuse is higher. In addition, the resin of the above type is lower in cost. Based on this, the carrier of the present disclosure is better in enzyme compatibility and better in immobilization effect, and the activity of the enzyme can be reserved to a great extent during immobilization. Therefore, the immobilized enzyme obtained through immobilization is better in activity and higher in stability during reuse, and is more suitable for application in industrialized immobilized enzymes.

In conclusion, the present disclosure effectively solves the problems in the related art that an enzyme immobilization carrier is relatively poor in enzyme compatibility and poor in immobilization effect, and an immobilized enzyme is relatively poor in activity, and relatively poor in stability during repeated use.

Preferably, the amino-type methacrylic resin has an amino functional group with a C2 or C6 length carbon chain arm, and a content of the amino is 30-80 µmol/g. Preferably, the amino-type methacrylic resin is one or more of Seplite^{®}LX-1000HA, Seplite^{®}LX-1000EPN, Seplite^{®}LX-EPHA, Seplite^{®}LX-1000EA, Lifetech^{™}ECR8309, Lifetech^{™}ECR8409, ESR-1, ESR-3, or ESQ-1. More preferably, the amino-type methacrylic resin is one or more of Seplite^{®}LX-1000HA, Seplite^{®} LX-1000EPN, Seplite^{®}LX-EPHA, or Lifetech^{™}ECR8309.

Preferably, the epoxy-type methacrylic resin has an epoxide equivalent of 2-5 µmol/g. More preferably, the epoxy-type methacrylic resin is one or more of Seplite^{®}LX-1000EP, Seplite^{®}LX-103B, EP200, Seplite^{®}LX-107B, Seplite^{®}LX-1000SW, Seplite^{®}LX-1000SD, Seplite^{®}LX-109s, Seplite^{®} LX-1000HFA, Lifetech^{™}ECR8285, Lifetech^{™}ECR8204, Lifetech^{™}ECR8209, ES-1, ES103, ES-101, ReliZyme^{™}HFA403, or ReliZyme^{™}EC-HFA. More preferably, the epoxy-type methacrylic resin is one or more of Seplite ^{®} LX-1000EP, Seplite ^{®} LX-109s, Seplite ^{®} LX-1000HFA, or ReliZyme^{™}EC-HFA.

The resin ball matrix is selected from the above types. On the one hand, the resin ball matrix is higher in mechanical strength, larger in particle than that of the traditional affinity resin, and lower in cost, such that the resin ball matrix is more liable to apply on a large scale, and has better prospects in industrial production. On the other hand, the formed carrier is better in enzyme compatibility, the activity of the enzyme can be reserved to a great extent during immobilization, and the stability of the enzyme during reuse is higher. Sources of the resins are shown below:

| Brand | Resin name | Matrix | Functional group |
|---|---|---|---|
| SUNRESIN | LX-1000HA | Methyl propiolate | Long chain amino (C6) |
| SUNRESIN | LX-1000EPN | Methyl propiolate | Short chain amino (C2) |
| SUNRESIN | LX-1000EA | Methyl propiolate | Short chain amino (C2) |
| SUNRESIN | LX-EPHA | Methyl propiolate | Short chain amino (C2) |
| PUROLITE | ECR8309 | Methyl propiolate | Short chain amino (C2) |
| PUROLITE | ECR8409 | Methyl propiolate | Long chain amino (C6) |
| NANKAI HECHENG | ESR-1 | Methyl propiolate | Amino (hydrophilic) |
| NANKAI HECHENG | ESR-3 | Methyl propiolate | Amino (hydrophobic) |
| NANKAI HECHENG | ESQ-1 | Methyl propiolate | Amino (hydrophobic) |
| SUNRESIN | LX-1000EP | Methyl propiolate | Epoxy group |
| SUNRESIN | LX-103B | Methyl propiolate | Epoxy group |
| SUNRESIN | EP200 | Methyl propiolate | Epoxy group |
| SUNRESIN | LX-107B | Methyl propiolate | Epoxy group |
| SUNRESIN | LX-1000SW | Methyl propiolate | Epoxy group |
| SUNRESIN | LX-1000SD | Methyl propiolate | Epoxy group |
| SUNRESIN | LX-109s | Methyl propiolate | Epoxy group |
| SUNRESIN | LX-1000HFA | Methyl propiolate | Amino-epoxy group |
| PUROLITE | ECR8285 | Methyl propiolate | Epoxy group |
| PUROLITE | ECR8204 | Methyl propiolate | Epoxy group |
| PUROLITE | ECR8209 | Methyl propiolate | Epoxy group |
| NANKAI HECHENG | ES-1 | Methyl propiolate | Epoxy group (hydrophilic) |
| NANKAI HECHENG | ES103 | Methyl propiolate | Epoxy group (hydrophobic) |
| MITSUBISHI CHEMICAL | ES-101 | Methyl propiolate | Epoxy group (hydrophobic) |
| MITSUBISHI CHEMICAL | HFA403 | Methyl propiolate | Epoxy group |
| MITSUBISHI CHEMICAL | EC-HFA | Methyl propiolate | Epoxy group |

In order to further improve the bonding stability of the enzyme and the carrier, preferably, the metal ion is a nickel ion, an iron ion, a copper ion, or a cobalt ion; and more preferably, the metal ion is the nickel ion, the copper ion, or the cobalt ion.

The present disclosure further provides an immobilized enzyme. The immobilized enzyme includes the enzyme immobilization carrier, and an enzyme immobilized thereon.

Based on various reasons mentioned earlier, the carrier of the present disclosure is better in enzyme compatibility and better in immobilization effect, and the activity of the enzyme can be reserved to a great extent during immobilization. Therefore, the immobilized enzyme obtained through immobilization is better in activity and higher in stability during reuse, and is more suitable for application in industrialized immobilized enzymes.

The immobilized enzyme of the present disclosure has wide applicability to the enzymes. For example, the enzyme includes, but is not limited to, any one or more of a transaminase, a ketoreductase, an alcohol dehydrogenase, a FDH, a glucose dehydrogenase, a monooxygenase, an ene-reductase, an imine reductase, and an amino acid dehydrogenase. It is more suitable for the following enzymes, the transaminase is a transaminase derived from *Chromobacterium violaceum DSM30191,* or a transaminase derived from *Arthrobacter citreus,* or a transaminase derived from *Actinobacteria,* or a transaminase derived from *Sciscionella sp. SE31;* the ketoreductase is a carbonyl reductase derived from *Acetobacter sp.CCTCC M209061,* or a ketoreductase derived from *Sporobolomyces salmonicolor,* the alcohol dehydrogenase is derived from *Thermoanaerobium brockii;* the FDH is a FDH derived from Candida boidinii; the glucose dehydrogenase is a glucose dehydrogenase derived from *Lysinibacillus sphaericus G10;* the monooxygenase is a cyclohexanone monooxygenase derived from *Rhodococcus sp.Phi1*, or a cyclohexanone monooxygenase derived from *Rhodococcus ruber-SD1*, or a cyclohexanone monooxygenase derived from *Brachymonas petroleovorans;* the ene-reductase is an ene-reductase derived from *Saccharomyces cerevisiae,* or an ene-reductase derived from *Chryseobacterium sp.CA49;* the imine reductase is an imine reductase derived from *Streptomyces sp.,* or an imine reductase derived from *Bacillus cereus;* and the amino acid dehydrogenase is a leucine dehydrogenase derived from *Bacillus cereus,* or a phenylalanine dehydrogenase derived from *Bacillus sphaericus;* or an amino acid dehydrogenase derived from *Thermoactinomyces intermedius ATCC33205,* or an amino acid dehydrogenase derived from *Thermosyntropha lipolytica.* The enzyme numbers, sources, and enzyme sequence information for the above enzymes of the present disclosure are shown below:

| Enzym e | Enzyme number | Source | Enzyme sequence information |
|---|---|---|---|
| Transa minase | TA-Cv | Transaminase from *Chromobacterium violaceum DSM30191* | SEQ ID NO: 1 (CN108048419B) |
| | TA-Cv-1 | | SEQ ID NO: 2+R416T+T7C+S47C+R405E+K90G+A95 P+K304D+Q380L+I297L (CN108384767B) |
| | TA-Ac | ω-Transaminase from *Arthrobacter citreus* | SEQ ID NO: 3 (CN110205310A) |
| | TA-Ac-1 | | SEQ ID NO: 3+Ars71+K7N+E424Q (CN 110205310A) |
| | TA-Ac-2 | | SEQ ID NO: 3+Ars76+E424Q (CN110205310A) |
| | TA-Ac-3 | | SEQ ID NO: 3+L3S+V5S+C60Y+F164L+A178L+S187A +I180V+L370A+G411D+S186G+Y384F+I 389F+V252I+L404Q+E171D (CN110205310A) |
| | TA IV-Ac | Aminotransferase IV from *Actinobacteria* | SEQ ID NO: 4 (CN107828751B) |
| | TA IV-Ac-1 | | SEQ ID NO: 4+L76A&S125A&A226G&S132A (CN107828751B) |
| | TA IV-Ac-2 | | SEQ ID NO: 4+L107I+L166I+A168I+KI49Y+H73N (CN107828751B) |
| | TA IV-Ac-3 | | SEQ ID NO: 4+L107I+L166I+A168I+K149H+K146R (CN107828751B) |
| | TA IV-Ss | Aminotransferase IV from *Sciscionella sp. SE31* | SEQ ID NO: 5 (CN110628742A) |
| | TA IV-Ss-1 | | SEQ ID NO: 5+G69Y+H70T+L73A+V77G+A78I+Y130 V+K141S+S142T+R143P+G144Y+L148A +L151A+T152R+L163Q+A165I+S207I+F2 08R+K211H+T290S+A292G+12 amino acids increased in N-termal +F11I+G17V+Q40H+T204S+T66M+A151 E+V130M (CN110628742A) |
| | TA IV-Ss-2 | | SEQ ID NO: 5+G69Y+H70T+L73A+V77G+A78I+Y130 V+K141S+S142T+R143P+G144Y+L148A +L151A+T152R+L163Q+A165I+S207I+F2 08R+K211H+T290S+A292G+12 amino acids increased in N-termal +F11I+G17V+Q40H+T204S+T66M+A151 E+V130M (CN110628742A) |
| | TA IV-Ss-3 | | SEQ ID NO:5+G17V+Q40H+ T66M+G69Y+H70T + L73A+V77G+A78I+Y130V+K141S+S142T +R143P+G144Y+L148A+L151E+T152R+ L163Q+A165I+T204S+S2071+F208R+K21 1H+T290S+A292G+12 amino acids increased in N-termal +F-11I+E151H+H153P+E145G+K146R+Y 198F+V130M+S204N+S278R+V244H (CN110628742A) |
| Ketore ductas e | CR-Ac | Carbonyl reductase from *Acetobacter sp. CCTCC M209061* | SEQ ID NO: 6 (CN108048417B) |
| | CR-Ac-1 | | SEQ ID NO: 6+E144S+A94N+N156V (CN108048417B) |
| | CR-Ac-2 | | SEQ ID NO: 6+E144A+L152Y+L198Q+E201G+G6S+L 146M+I147V+D42E+T199V+A21V+A58T+ S96N (CN108048417B) |
| Alcohol dehydr ogenas e | ADH-Tb | Alcohol Dehydrogenase from *Thermoanaerobium brockii* | ABY93890.1 |
| Ketore ductas e | KRED-Ss | Ketoreductase from *Sporobolomyces salmonicolor* | Q9UUN9.3 |
| FDH | FDH | Formate Dehydrogenase from *Candida boidinii* | AIY34662.1 |
| Glucos e dehydr ogenas e | GDH | Glucose 1-dehydrogenase from *Lysinibacillus sphaericus G10* | ACR78513.1 |
| Cycloh exanon e monoo | CHMO-Rr | Cyclohexanone monooxygenase from *Rhodococcus ruber-SD1* | SEQ ID NO: 7 (CN109402074A) |
| | CHMO-Rr-1 | | SEQ ID NO .7+P190L+Y559M+C249V+C393V+C2 57A+M45T (CN109402074A) |
| xygena se | CHMO-Rr-2 | | SEQ ID NO .7+P190I+Y560F (CN109402074A) |
| | CHMO-Rs | Cyclohexanone monooxygenase from *Rhodococcus sp. Phi1* | SEQ ID NO: 8 (CN110055230A) |
| | CHMO-Rs -1 | | SEQ ID NO: 8+F508Y+A435N+L438A+A436S+F280V+ S441V+L510V (CN110055230A) |
| | CHMO-Bp | Cyclohexanone monooxygenase from *Brachymonas petroleovorans* | SEQ ID NO: 9 (CN108300707B) |
| Ene-re ductas e | ERED-Chr | Old yellow enzyme from *Chryseobacterium sp. CA49* | ALE60336.1 |
| | ERED-Sc | *Saccharomyces cerevisiae* | AJU28279.1 |
| Imine reducta se | IRED-Str | Imine Reductase from *Streptomyces sp.* | WP_086772432 |
| | IRED-Bc | Imine reductase from *Bacillus cereus* | WP_095755701.1 |
| Amino acid dehydr ogenas e | AADH-Bc | Leucine Dehydrogenase from *Bacillus cereus* | WP_000171355.1 |
| | AADH-Bs | Phenylalanine Dehydrogenase from *Bacillus sphaericus* | P23307.1 |
| | AADH-Ti | Thermostable Phenylalanine dehydrogenase (TIPDH) from *Thermoactinomyces intermedius ATCC33205* | SEQ ID NO: 10 (CN108795893A) |
| | AADH-Ti-1 | | SEQ ID NO:10+A135L (CN108795893A) |
| | AADH-Ti-2 | | SEQ ID NO:10+ K144G+L294I (CN108795893A) |
| | AADH-TL | diaminopimelate dehydrogenase from *Thermosyntropha lipolytica* | SEQ ID NO: 11 (CN109022381A) |
| | AADH-TL-1 | | SEQ ID NO: 11+R183C-H229S (CN109022381A) |
| | AADH-TL-2 | | SEQ ID NO: 11+R183C-H229S-Y207R (CN109022381A) |

It is to be noted that, the above enzyme sequences are all disclosed enzymes in the related art, and the present disclosure only uses the above enzymes for the verification of the performance of the enzyme immobilization carrier of the present disclosure.

In order to further improve the activity and reuse stability of the immobilized enzyme, more preferably, when the enzyme is the transaminase, the matrix resin is one or more of LX-1000HA, LX-1000EPN, LX-EPHA, or LX-109s; when the enzyme is the FDH, the matrix resin is one or more of LX-1000EPN, LX-109s, LX-1000HFA, ECR8285, or EC-HFA; when the enzyme is the ketoreductase, the matrix resin is one or more of LX-109s, LX-EPHA, ECR8285, LX-1000HA, or ECR8409; when the enzyme is the monooxygenase, the matrix resin is one or more of LX-109s, LX-EPHA, or LX-1000HA; when the enzyme is the imine reductase, the matrix resin is LX-109s and/or LX-EPHA; and when the enzyme is the amino acid dehydrogenase, the alcohol dehydrogenase, or the ene-reductase, the matrix resin is one or more of LX-109s, LX-1000EPN, LX-EPHA, or LX-1000HA.

The present disclosure further provides a method for preparing the enzyme immobilization carrier. The method includes the following steps: providing a resin ball matrix; linking an -N(CH₂COOH)₂ group onto the resin ball matrix by means of a chemical bond; absorbing a metal ion onto the -N(CH₂COOH)₂ group in a chelating manner through coordination, so as to obtain the enzyme immobilization carrier.

Based on various reasons mentioned earlier, the carrier of the present disclosure is better in enzyme compatibility and better in immobilization effect, and the activity of the enzyme can be reserved to a great extent during immobilization. Therefore, the immobilized enzyme obtained through immobilization is better in activity and higher in stability during reuse, and is more suitable for application in industrialized immobilized enzymes.

In a preferred implementation solution, when the resin ball matrix is the amino-type methacrylic resin, the amino-type methacrylic resin is mixed with sodium chloroacetate for nucleophilic substitution, so as to link the -N(CH₂COOH)₂ group onto the amino-type methacrylic resin. More preferably, after an aqueous solution of the amino-type methacrylic resin and the sodium chloroacetate is stirred for 30-60 min, the reaction system is regulated to a pH of 9-10 with 1-2 mol/L of an aqueous alkaline solution, and then heated up to 70-80 °C for reaction for 20-30 h at an N₂ atmosphere, so as to link the -N(CH₂COOH)₂ group onto the amino-type methacrylic resin. Preferably, the aqueous alkali solution is a sodium carbonate solution, a dilute sodium hydroxide solution, or a lithium hydroxide solution. Based on this, the amino-type methacrylic resin ball matrix of the present disclosure is higher in conversion rate, and more stable in reaction process.

In a preferred implementation solution, when the resin ball matrix is the epoxy-type methacrylic resin, the epoxy-type methacrylic resin is mixed with Iminodiacetic acid disodium for an addition reaction, so as to link the -N(CH₂COOH)₂ group onto the epoxy-type methacrylic resin. More preferably, after an aqueous solution of the epoxy-type methacrylic resin and the Iminodiacetic acid disodium is stirred for 30-60 min at 20-25 °C, the reaction system is heated up to 60-70 °C for reaction for 18-24 h at an N₂ atmosphere, so as to link the -N(CH₂COOH)₂ group onto the epoxy-type methacrylic resin. Based on this, the epoxy-type methacrylic resin ball matrix of the present disclosure is higher in conversion rate, and more stable in reaction process.

In a preferred implementation solution, after the step of linking the -N(CH₂COOH)₂ group onto the resin ball matrix by means of the chemical bond, a metal salt solution is added to the reaction system for a complex reaction, so as to absorb the metal ion on the -N(CH₂COOH)₂ group in the chelating manner through coordination, thereby obtaining the enzyme immobilization carrier. Preferably, during the complex reaction, a reaction temperature is 2-30 °C and reaction time is 1-4h; and the metal salt solution is an aqueous solution of nickel chloride, an aqueous solution of copper sulfate, an aqueous solution of ferrous chloride, or an aqueous solution of cobalt chloride. Based on this, the reaction condition is more appropriate, such that the enzyme immobilization carrier obtained through reaction is higher in yield and higher in purity. More preferably, in the metal salt solution, a mass ratio of the metal ion to the resin ball matrix is (0.05-0.1):1.

In order to further improve the conversion rate of the amino-type matrix resin, preferably, a mass ratio of the sodium chloroacetate to the amino-type methacrylic resin ball is (5-10):1.

In order to further improve the conversion rate of the epoxy-type matrix resin, preferably, a mass ratio of the Iminodiacetic acid disodium to the epoxy-type methacrylic resin ball is (5-10):1.

The present disclosure further provides a method for preparing the immobilized enzyme. The method includes the following step: performing an immobilization reaction on the enzyme immobilization carrier, and an enzyme, so as to obtain the immobilized enzyme.

Based on various reasons mentioned earlier, the carrier of the present disclosure is better in enzyme compatibility and better in immobilization effect, and the activity of the enzyme can be reserved to a great extent during immobilization. Therefore, the immobilized enzyme obtained through immobilization is better in activity and higher in stability during reuse, and is more suitable for application in industrialized immobilized enzymes.

Preferably, the method for preparing the immobilized enzyme includes the following step: dispersing the enzyme immobilization carrier into a first solvent to form dispersion liquid, where the first solvent is a mixed solution of a phosphate buffer solution, a sodium chloride aqueous solution, and an imidazole buffer solution; and reacting the dispersion liquid with an enzyme solution containing the enzyme, to cause the enzyme and the enzyme immobilization carrier to be subjected to an immobilization reaction, so as to obtain the immobilized enzyme. The first solvent creates a milder and more appropriate environment for immobilization, such that the activity of the enzyme may be protected, an enzyme immobilization effect is improved, and non-target proteins in an enzyme solution are prevented from being immobilized. Based on this, the enzyme compatibility of the carrier is further improved, and the enzyme immobilization effect is better, and the activity is better.

In order to further improve the activity and reuse stability of the immobilized enzyme, preferably, in the first solvent, the phosphate buffer solution has the concentration of 0.1-0.2 mol/L; preferably, the sodium chloride aqueous solution has the concentration of 0.5-1 mol/L; and preferably, the imidazole buffer solution has the concentration of 0.05-0.1 mol/L.

In order to improve the stability during reaction while improving reaction efficiency, preferably, during the reaction process of the dispersion liquid and the enzyme solution, a reaction temperature is 20-25 °C, and reaction time is 16-24h. More preferably, per gram of the enzyme immobilization carrier corresponds to 4-8 mL of the enzyme solution, and the enzyme solution has protein content of 20-25 mg/mL. Therefore, the enzyme is immobilized more fully, a carrying capacity is provided, and the immobilized enzyme has higher catalytic activity.

The present application is further described in detail below with reference to specific embodiments, and the embodiments cannot be construed as limiting the scope of protection claimed in the present application.

### Embodiment 1

Immobilization and application of a transaminase on an enzyme immobilization carrier.

Preparation of the enzyme immobilization carrier by using an amino-type methacrylic resin as a resin ball matrix:
5 g of the amino-type methacrylic resin ball matrix was taken and added to a 250 mL four-necked bottle; 75 mL of deionized water and 22.5 g of sodium chloroacetate were added; mechanical stirring was performed at 120 rpm at room temperature, so as to uniformly disperse the carrier; after 30 min of stirring, pH was regulated to 9-10 with a 1 MNa₂CO₃ solution; and then the mixture was heated to 70°C for reaction for 20h under N₂ protection (a pH value was regulated for a plurality of times through the process, so as to cause the pH value to keep at 9-10). After the reaction ended, the system was cooled to room temperature and washed to neutral with water. Liquid in the system was removed; after modification, the carrier was washed twice with the deionized water; 100 mL of a 5mol/L NiCl aqueous solution was added; stirring was performed for 3h; a metal ion system was removed; washing was performed twice with the deionized water.

Preparation of the enzyme immobilization carrier by using an epoxy-type methacrylic resin as a resin ball matrix:
5 g of the epoxy-type methacrylic resin ball matrix was taken and added to a 250 mL four-necked bottle; 75 mL of a 2M hydrated Iminodiacetic acid disodium salt solution was added; mechanical stirring was performed at 120 rpm at room temperature, so as to uniformly disperse the carrier; after 30 min of stirring, the mixture was heated to 60 °C for reaction for 18h under N₂ protection. After the reaction ended, the system was cooled to room temperature and washed to neutral with water. Liquid in the system was removed; after modification, the carrier was washed twice with the deionized water; 100 mL of a 5 mmol/L NiCl aqueous solution was added; stirring was performed for 3h; a metal ion system was removed; washing was performed twice with the deionized water.

Immobilization of the transaminase on the enzyme immobilization carrier using the amino-type methacrylic resin as the resin ball matrix:
1 g of the enzyme immobilization carrier was weighed, and washed for a plurality of times by using a 0.1M phosphate buffer solution (pH7.0), 0.5M NaCl, and a 0.05M imidazole buffer solution; and the buffer solutions were removed, and the carrier was maintained to be used. Then 4 mL of an enzyme solution (the enzyme solution was prepared by using the 0.2M phosphate buffer solution (pH7.0), 0.8M NaCl, and the 0.05M imidazole buffer solution, so as to cause the protein content to be 20-25 mg/mL, and had a cofactor PLP), incubation was performed for 16-24h at 20 °C, and the buffer solutions were removed. Washing was performed for 3 times by using a 20M phosphate buffer solution (pH8.0) containing 0.5M NaCl and the 0.05M imidazole buffer solution, and then was performed once by using a 0.1M phosphate buffer solution (pH7.0) and a 0.5M NaCl buffer solution; the buffer solutions were removed; and the immobilized enzyme was maintained to be used.

Immobilization of the transaminase on the enzyme immobilization carrier using the epoxy-type methacrylic resin as the resin ball matrix:
immobilization of the transaminase on the epoxy-type methacrylic resin: 1 g of an epoxy-type resin was weighed, and washed for a plurality of times by using the 0.2M phosphate buffer solution (pH7.0) and a 0.8M NaCl buffer solution; the buffer solutions were removed; and the resin was maintained to be used. Then 4 mL of an enzyme solution (having the cofactor pyridoxal phosphate PLP), which was prepared by using the 0.2M phosphate buffer solution (pH7.0), the 0.8M NaCl buffer solution, and 0.1 g of enzyme powder, was added; incubation was performed for 36-48h at 20 °C, and the buffer solutions were removed. Washing was performed for 3 times by using the 0.1M phosphate buffer solution (pH7.0) and the 0.5M NaCl buffer solution; the buffer solutions were removed; and the immobilized enzyme was maintained to be used.

Immobilization of the transaminase on the amino-type methacrylic resin: 1 g of an amino-type resin was weighed, and washed for a plurality of times by using the 0.1M phosphate buffer solution 7.0; the buffer solution was removed; and the 0.1M phosphate buffer solution (pH7.0), and a glutaraldehyde solution with a final concentration being 2% were added. Then 4 mL of an enzyme solution (having the cofactor PLP), which was prepared by using the 0.1M phosphate buffer solution 7.0 and 0.1 g of the enzyme powder, was added; incubation was performed for 16-24h at 20 °C, and the buffer solution was removed. Washing was performed for 3 times by using the 20M phosphate buffer solution 7.0 containing 0.5M NaCl; the buffer solution was removed; and the immobilized enzyme was maintained to be used.

Immobilization of the transaminase on a commercial affinity chromatography resin in the related art:

| Brand | Resin name | Matrix | Functional group |
|---|---|---|---|
| PUROLITE | MIDA-Ni | Methyl propiolate | Ni+ |
| Bio-Rad | IMAC-Ni | Methyl propiolate | Ni+ |

1 g of a Bio-Rad IMAC-Ni or PUROLITE MIDA-Ni immobilized carrier was weighed, and washed for a plurality of times by using the 0.1M phosphate buffer solution (pH7.0), the 0.5M NaCl, and the 0.05M imidazole buffer solution; and the buffer solutions were removed, and the carrier was maintained to be used. Then 4 mL of an enzyme solution (the enzyme solution was prepared by using the 0.2M phosphate buffer solution (pH7.0), 0.8M NaCl, and the 0.05M imidazole buffer solution, so as to cause the protein content to be 20-25 mg/mL, and had a cofactor PLP), incubation was performed for 16-24h at 20 °C, and the buffer solutions were removed. Washing was performed for 3 times by using a 20M phosphate buffer solution (pH8.0) containing 0.5M NaCl and the 0.05M imidazole buffer solution, and then was performed once by using a 0.1M phosphate buffer solution (pH7.0) and a 0.5M NaCl buffer solution; the buffer solutions were removed; and the immobilized enzyme was maintained to be used.

Immobilized transaminase activity and reusability test:

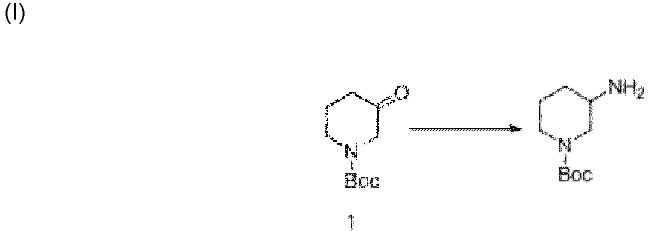

0.1 g of a main raw material 1 was added to a 10 mL reaction bottle; 4eq isopropylamine hydrochloride and 1 mg of the PLP were added; the 0.1M phosphate buffer solution 7.0 was replenished to make the final volume of the reaction solution up to 1 mL; then 0.01 g of the enzyme powder or the immobilized enzyme, which was prepared by 0.01 g of the enzyme powder, was added; and stirring was performed for 16-20h at 30 °C. A conversion rate was tested. At the end of each round of the reaction, the immobilized enzyme was separated and reused in the next reaction. The number of reuse was checked. The conversion rate of the system was detected through HPLC. reaction data was shown in Table 1 below:

**Table 1**

| Enzyme | Resin name | Conversion rate | Number of cycles |
|---|---|---|---|
| TA-Cv | Resin-free free enzyme | >98% | 1 |
| | LX-1000HA-IDA-Ni | >90% | 15 |
| | LX-1000EPN-IDA-Ni | >96% | 18 |
| | LX-EPHA-IDA-Ni | >90% | 17 |
| | LX109s-IDA-Ni | >90% | 14 |
| | LX-1000HA | >80% | 9 |
| | LX-1000EPN | >80% | 8 |
| | LX-1000EA | >80% | 3 |
| | LX-EPHA | >80% | 7 |
| | LX109s | >80% | 6 |
| | MIDA-Ni | >90% | 6 |
| | IMAC-Ni | >90% | 8 |
| TA-Cv-1 | Resin-free free enzyme | >98% | 1 |
| | LX-1000HA-IDA-Ni | >96% | 18 |
| | LX-1000EPN-IDA-Ni | >98% | 21 |
| | LX-EPHA-IDA-Ni | >96% | 16 |
| | LX109s-IDA-Ni | >96% | 15 |
| | LX-1000HA | >90% | 8 |
| | LX-1000EPN | >90% | 7 |
| | LX-EPHA | >90% | 6 |
| | LX109s | >90% | 6 |
| | MIDA-Ni | >90% | 12 |
| | IMAC-Ni | >90% | 12 |
| TA-Ac | Resin-free free enzyme | >99% | 1 |
| | LX-1000HA-IDA-Ni | >98% | 8 |
| | LX-1000EPN-IDA-Ni | >98% | 12 |
| | LX-EPHA-IDA-Ni | >98% | 9 |
| | LX109s-IDA-Ni | >98% | 10 |
| | LX-1000HA | >90% | 6 |
| | LX-1000EPN | >90% | 5 |
| | LX-EPHA | >90% | 6 |
| | LX109s | >90% | 7 |
| | MIDA-Ni | >90% | 6 |
| | IMAC-Ni | >90% | 6 |
| TA-Ac-1 | Resin-free free enzyme | >99% | 1 |
| | LX-1000HA-IDA-Ni | >98% | 12 |
| | LX-1000EPN-IDA-Ni | >98% | 15 |
| | LX-EPHA-IDA-Ni | >98% | 9 |
| | LX109s-IDA-Ni | >98% | 10 |
| | LX-1000HA | >90% | 8 |
| | LX-1000EPN | >90% | 6 |
| | LX-EPHA | >90% | 6 |
| | LX109s | >90% | 5 |
| | MIDA-Ni | >90% | 8 |
| | IMAC-Ni | >90% | 7 |
| TA-Ac-2 | Resin-free free enzyme | >99% | 1 |
| | LX-1000HA-IDA-Ni | >98% | 15 |
| | LX-1000EPN-IDA-Ni | >98% | 11 |
| | LX-1000HA | >90% | 7 |
| | LX-1000EPN | >90% | 7 |
| | MIDA-Ni | >90% | 8 |
| | IMAC-Ni | >90% | 9 |
| TA-Ac-3 | Resin-free free enzyme | >99% | 1 |
| | LX-1000HA-IDA-Ni | >98% | 17 |
| | LX-1000EPN-IDA-Ni | >98% | 14 |
| | LX-1000HA | >90% | 7 |
| | LX-1000EPN | >90% | 7 |
| | MIDA-Ni | >90% | 10 |
| | IMAC-Ni | >90% | 12 |

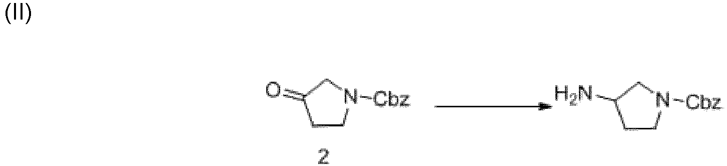

0.05 g of Dimethyl Sulfoxide (DMSO) was added in a 10 mL reaction bottle; 0.03 g of a main raw material 2 was dissolved; 3eq isopropylamine hydrochloride and 1 mg of the PLP were added; the 0.1M phosphate buffer solution (pH7.0) was replenished to make the final volume of the reaction solution up to 1 mL; then 0.01 g of the enzyme powder or the immobilized enzyme, which was prepared by 0.01 g of the enzyme powder, was added; and stirring was performed for 16-20h at 30 °C. A conversion rate was tested. At the end of each round of the reaction, the immobilized enzyme was separated and reused in the next reaction. The number of reuse was checked. The conversion rate of the system was detected through HPLC. reaction data was shown in Table 2 below:

**Table 2**

| | | | |
|---|---|---|---|
| TA-Ac | Resin-free free enzyme | >96% | 1 |
| | LX-1000HA-IDA-Ni | >90% | 7 |
| | LX-1000EPN-IDA-Ni | >90% | 11 |
| | LX-EPHA-IDA-Ni | >90% | 9 |
| | LX109s-IDA-Ni | >90% | 10 |
| | LX-1000HA | >80% | 6 |
| | LX-1000EPN | >80% | 5 |
| | LX-EPHA | >80% | 6 |
| | LX109s | >80% | 7 |
| | MIDA-Ni | >80% | 6 |
| | IMAC-Ni | >80% | 6 |
| TA-Ac-1 | Resin-free free enzyme | >96% | 1 |
| | LX-1000HA-IDA-Ni | >90% | 10 |
| | LX-1000EPN-IDA-Ni | >90% | 15 |
| | LX-EPHA-IDA-Ni | >90% | 11 |
| | LX109s-IDA-Ni | >90% | 16 |
| | LX-1000HA | >80% | 8 |
| | LX-1000EPN | >80% | 5 |
| | LX-EPHA | >80% | 6 |
| | LX109s | >80% | 7 |
| | MIDA-Ni | >80% | 5 |
| | IMAC-Ni | >80% | 7 |
| TA-Ac-2 | Resin-free free enzyme | >96% | 1 |
| | LX-1000HA-IDA-Ni | >90% | 13 |
| | LX-109s-IDA-Ni | >90% | 11 |
| | LX-1000HA | >80% | 7 |
| | LX-109s | >80% | 7 |
| | MIDA-Ni | >80% | 8 |
| | IMAC-Ni | >80% | 6 |
| TA-Ac-3 | Resin-free free enzyme | >96% | 1 |
| | LX-1000HA-IDA-Ni | >90% | 17 |
| | LX-109s-IDA-Ni | >90% | 14 |
| | LX-1000HA | >80% | 7 |
| | LX-109s | >80% | 7 |
| | MIDA-Ni | >80% | 10 |
| | IMAC-Ni | >80% | 7 |

### Embodiment 2

Immobilization and application of a FDH on an enzyme immobilization carrier.

The difference between this embodiment and Embodiment 1 lied in that, the transaminase was replaced with the FDH with equal mass, and the cofactor was replaced with NAD⁺ with equal mass.

Immobilized FDH activity and reusability test:

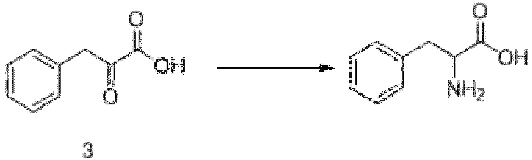

5 mL of a 0.1MTris-Cl buffer solution (pH8.0-9.0) was added to a 50 mL reaction bottle; 100 mg of a main raw material 3 was dissolved; 108 mg of ammonium chloride, and 80 mg of ammonium salt were added to regulate the pH to be 7.5-8.0; then 10 mg of the NAD⁺, 100 mg of an AADH-Bc free enzyme, and 5 mg of FDH enzyme powder or immobilized FDH, which is prepared by 5 mg of the enzyme powder, were added. Stirring was performed for 16-20h at 30 °C. At the end of each round of the reaction, the immobilized enzyme was separated and reused in the next reaction. The number of reuse was checked. The conversion rate of the system was detected through HPLC. reaction data was shown in Table 3 below:

**Table 3**

| Enzyme | Resin name | Conversion rate | Number of cycles |
|---|---|---|---|
| FDH | Resin-free free enzyme | 99.9% | 1 |
| | FDH-free free enzyme | 0% | 1 |
| | LX-1000EP-IDA-Ni | >90% | 12 |
| | LX-109s-IDA-Ni | >98% | 18 |
| | LX-1000HFA-IDA-Ni | >90% | 22 |
| | ECR8285-IDA-Ni | >90% | 11 |
| | EC-HFA-IDA-Ni | >90% | 12 |
| | LX-1000EP | >80% | 4 |
| | LX-1000SD | >80% | 3 |
| | LX-109s | >80% | 4 |
| | LX-1000HFA | >80% | 4 |
| | MIDA-Ni | >90% | 7 |
| | IMAC-Ni | >90% | 6 |

### Embodiment 3

Immobilization and application of a ketoreductase on an enzyme immobilization carrier.

The difference between this embodiment and Embodiment 1 lied in that, the transaminase was equivalently replaced with the ketoreductase, and the cofactor was replaced with NAD⁺ with equal mass.

Immobilized ketoreductase activity and reusability test:

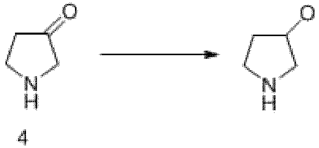

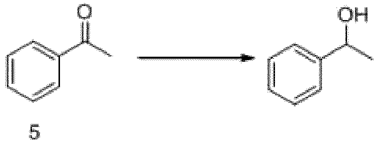

0.5 mL of Iso-Propyl Alcohol (IPA) was added in a 10 mL reaction bottle; 0.1g of a raw material 4 or 5 was dissolved; 0.5 mL of a 0.1M phosphate buffer solution 7.0 and 10 mg of NAD⁺ were added; then 10 mg of enzyme powder or an immobilized enzyme, which was prepared by 10 mg of the enzyme powder, was added; and stirring was performed for 16-20h at 30 °C. At the end of each round of the reaction, the immobilized enzyme was separated and reused in the next reaction. The number of reuse was checked. Reaction data was shown in Table 4 below:

**Table 4**

| Enzyme | Resin name | Raw material 4 | | Raw material 5 | |
|---|---|---|---|---|---|
| | | Conversion rate | Number of cycles | Conversion rate | Number of cycles |
| KRED-Ss | Resin-free free enzyme | 99.1% | 1 | 96.3% | 1 |
| | LX-109s-IDA-Ni | >90% | 12 | >90% | 12 |
| | LX-EPHA-IDA-Ni | >90% | 18 | >90% | 16 |
| | ECR8285-IDA-Ni | >90% | 11 | >90% | 10 |
| | ECR8409-IDA-Ni | >90% | 11 | >90% | 11 |
| | LX-109s | >80% | 2 | >90% | 2 |
| | LX-1000EA | >80% | 2 | >90% | 3 |
| | LX-EPHA | >80% | 3 | >90% | 3 |
| | MIDA-Ni | >90% | 4 | >90% | 5 |
| | IMAC-Ni | >90% | 3 | >90% | 6 |
| CR-Ac | Resin-free free enzyme | 99.4% | 1 | >97.1% | 1 |
| | LX-1000HA-IDA-Ni | >95% | 23 | >90% | 20 |
| | LX-109s-IDA-Ni | >95% | 20 | >90% | 21 |
| | LX-EPHA-IDA-Ni | >95% | 25 | >90% | 25 |
| | LX-1000HA | >90% | 12 | >90% | 7 |
| | LX-109s | >90% | 9 | >90% | 3 |
| | LX-EPHA | >90% | 13 | >90% | 2 |
| | MIDA-Ni | >90% | 15 | >90% | 7 |
| | IMAC-Ni | >90% | 13 | >90% | 5 |
| CR-Ac-1 | Resin-free free enzyme | 99.5% | 1 | >96.8% | 1 |
| | LX-1000HA-IDA-Ni | >95% | 25 | >90% | 25 |
| | LX-109s-IDA-Ni | >95% | 18 | >90% | 20 |
| | LX-EPHA-IDA-Ni | >95% | 23 | >90% | 23 |
| | LX-1000HA | >90% | 9 | >90% | 7 |
| | LX-109s | >90% | 7 | >90% | 4 |
| | LX-EPHA | >90% | 12 | >90% | 2 |
| | MIDA-Ni | >90% | 15 | >90% | 6 |
| | IMAC-Ni | >90% | 17 | >90% | 5 |
| CR-Ac-2 | Resin-free free enzyme | 99.8% | 1 | >98.2% | 1 |
| | LX-1000HA-IDA-Ni | >95% | 25 | >90% | 27 |
| | LX-109s-IDA-Ni | >95% | 21 | >90% | 23 |
| | LX-EPHA-IDA-Ni | >95% | 26 | >90% | 23 |
| | LX-1000HA | >90% | 10 | >90% | 6 |
| | LX-109s | >90% | 11 | >90% | 4 |
| | LX-EPHA | >90% | 10 | >90% | 3 |
| | MIDA-Ni | >90% | 13 | >90% | 5 |
| | IMAC-Ni | >90% | 13 | >90% | 6 |

### Embodiment 4

Immobilization and application of a monooxygenase on an enzyme immobilization carrier.

The difference between this embodiment and Embodiment 1 lied in that, the transaminase was equivalently replaced with the monooxygenase, and the cofactor was replaced with Nicotinamide Adenine Dinucleotide Phosphate (NADP⁺) with equal mass.

Immobilized monooxygenase activity and reusability test:

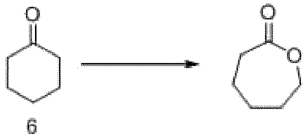

0.3 mL of IPA was added to a 10 ml reaction bottle; then 100 mg of a substrate 6, 5 mg of NADP⁺, 3 mL of a 0.1M phosphate buffer solution (pH8.0) were added; then 2 mg of an alcohol dehydrogenase ADH-Tb free enzyme and 20 mg of a cyclohexanone monooxygenase free enzyme or an immobilized enzyme prepared by 22 mg of the free enzyme were added. Reaction was performed for 16-20h at 30 °C. A conversion rate was tested. At the end of each round of the reaction, the immobilized enzyme was separated and reused in the next reaction. The number of reuse was checked. The conversion rate of the system was detected through gas chromatography. Reaction data was shown in Table 5 below:

**Table 5**

| Enzyme | Resin name | Conversion rate | Number of cycles |
|---|---|---|---|
| CHMO-Rr | Resin-free free enzyme | 99.1% | 1 |
| | LX-109s-IDA-Ni | >90% | 12 |
| | LX-EPHA-IDA-Ni | >90% | 14 |
| | LX-109s | >60% | 1 |
| | LX-EPHA | >60% | 1 |
| | MIDA-Ni | >90% | 3 |
| | IMAC-Ni | >90% | 3 |
| CHMO-Rr-1 | Resin-free free enzyme | 99.1% | 1 |
| | LX-1000HA-IDA-Ni | >90% | 13 |
| | LX-109s-IDA-Ni | >90% | 10 |
| | LX-EPHA-IDA-Ni | >90% | 15 |
| | LX-1000HA | >60% | 1 |
| | LX-109s | >60% | 1 |
| | LX-EPHA | >60% | 1 |
| | MIDA-Ni | >90% | 3 |
| | IMAC-Ni | >90% | 3 |
| CHMO-Rr-2 | Resin-free free enzyme | 99.3% | 1 |
| | LX-1000HA-IDA-Ni | >90% | 8 |
| | LX-109s-IDA-Ni | >90% | 10 |
| | LX-EPHA-IDA-Ni | >90% | 9 |
| | LX-1000HA | >60% | 1 |
| | LX-109s | >60% | 1 |
| | LX-EPHA | >60% | 1 |
| | MIDA-Ni | >90% | 2 |
| | IMAC-Ni | >90% | 3 |
| CHMO-Rs | Resin-free free enzyme | 98.2% | 1 |
| | LX-1000HA-IDA-Ni | >90% | 6 |
| | LX-109s-IDA-Ni | >90% | 5 |
| | LX-EPHA-IDA-Ni | >90% | 5 |
| | LX-1000HA | >60% | 1 |
| | LX-109s | >60% | 1 |
| | LX-EPHA | >60% | 1 |
| | MIDA-Ni | >90% | 3 |
| | IMAC-Ni | >90% | 2 |
| CHMO-Rs-1 | Resin-free free enzyme | 98.7% | 1 |
| | LX-1000HA-IDA-Ni | >90% | 10 |
| | LX-109s-IDA-Ni | >90% | 7 |
| | LX-EPHA-IDA-Ni | >90% | 8 |
| | LX-1000HA | >60% | 1 |
| | LX-109s | >60% | 1 |
| | LX-EPHA | >60% | 1 |
| | MIDA-Ni | >90% | 2 |
| | IMAC-Ni | >90% | 2 |

### Embodiment 5

Immobilization and application of an imine reductase on affinity-modified amino- and epoxy-type methacrylic resins.

The difference between this embodiment and Embodiment 1 lied in that, the transaminase was equivalently replaced with the imine reductase, and the cofactor was replaced with NAD⁺ with equal mass.

Immobilized imine reductase activity and reusability test:

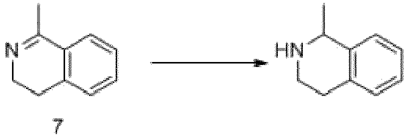

2 mL of a 0.1M phosphate buffer solution (pH7.0-8.0) was added to a 10 ml reaction ball; then 100 mg of the substrate, 10 mg of NAD⁺, 60 mg of ammonium formate, 10 mg of FDH, and 10 mg of an IRED free enzyme or an immobilized IRED enzyme prepared by 10 mg of the free enzyme or a co-immobilized enzyme prepared by 10 mg of the FDH and 10 mg of the IRED free enzyme were added. Reaction was performed for 20h at 30 °C. A conversion rate was tested. At the end of each round of the reaction, the immobilized enzyme was separated and reused in the next reaction. The number of reuse was checked. Test results of the conversion rate of the system that was detected through HPLC were shown in Table 6 below:

**Table 6**

| Enzyme | Resin name | Conversion rate | Number of cycles |
|---|---|---|---|
| IRED-Str | Resin-free free enzyme | 99.5% | 1 |
| | LX-109s-IDA-Ni | >90% | 7 |
| | LX-109s | >80% | 2 |
| | MIDA-Ni | >90% | 3 |
| | IMAC-Ni | >90% | 2 |
| IRED-Str+FDH | LX-109s-IDA-Ni | >90% | 10 |
| | LX-EPHA-IDA-Ni | >90% | 6 |
| | MIDA-Ni | >90% | 2 |
| IRED-Bc | Resin-free free enzyme | 99.6% | 1 |
| | LX-109s-IDA-Ni | >90% | 10 |
| | LX-EPHA-IDA-Ni | >90% | 7 |
| | LX-109s | >80% | 2 |
| | LX-EPHA | >80% | 2 |
| | MIDA-Ni | >90% | 2 |
| | IMAC-Ni | >90% | 4 |
| IRED-Bc+FDH | LX-109s-IDA-Ni | >90% | 10 |
| | LX-EPHA-IDA-Ni | >90% | 6 |
| | IMAC-Ni | >90% | 4 |

### Embodiment 6

Immobilization and application of Glutamic Dehydrogenase (GDH) on affinity-modified amino- and epoxy-type methacrylic resins.

The difference between this embodiment and Embodiment 1 lied in that, the transaminase was equivalently replaced with the GDH, and the cofactor was replaced with NADP⁺ with equal mass.

Immobilized GDH activity and reusability test:

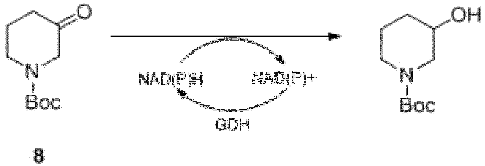

0.125 mL of methanol was added in a 10 mL reaction bottle; 0.25 g of a main raw material 8 was dissolved; 4eq glucose and 1 mg of the NADP⁺ were added; the 0.1M K phosphate buffer solution (pH7.2) was replenished to make the final volume of the reaction solution up to 2 mL; then 0.3 g of ADH-Tb enzyme powder and 10 mg of GDH enzyme powder or an immobilized enzyme prepared by 10 mg of the GDH enzyme powder were added; and stirring was performed for 20h at 35 °C. At the end of each round of the reaction, the immobilized enzyme was separated and reused in the next reaction. The number of reuse was checked. The conversion rate of the system was detected through HPLC. reaction data was shown in Table 7 below:

**Table 7**

| Enzyme | Resin name | Conversion rate | Number of cycles |
|---|---|---|---|
| GDH | Resin-free free enzyme | 98.7% | 1 |
| | LX-109s-IDA-Ni | >90% | 21 |
| | LX-1000EPN-IDA-Ni | >90% | 12 |
| | LX-EPHA-IDA-Ni | >90% | 17 |
| | LX-1000HA-IDA-Ni | >90% | 20 |
| | LX-109s | >90% | 8 |
| | LX-1000EPN | >90% | 7 |
| | LX-EPHA | >90% | 6 |
| | MIDA-Ni | >90% | 6 |
| | IMAC-Ni | >90% | 7 |

### Embodiment 7

Immobilization and application of an alcohol dehydrogenase on affinity-modified amino- and epoxy-type methacrylic resins.

The difference between this embodiment and Embodiment 1 lied in that, the transaminase was equivalently replaced with the alcohol dehydrogenase, and the cofactor was replaced with NADP⁺ with equal mass.

Immobilized alcohol dehydrogenase activity and reusability test:

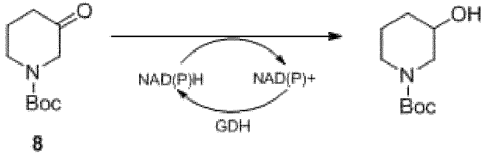

0.125 mL of methanol was added in a 10 mL reaction bottle; 0.25 g of a main raw material 8 was dissolved; 4eq glucose and 1 mg of the NADP⁺ were added; the 0.1M K phosphate buffer solution 7.2 was replenished to make the final volume of the reaction solution up to 2 mL; then 10 mg of GDH enzyme powder and 0.3 g of ADH enzyme powder, and/or an immobilized enzyme prepared by 0.3 g of the ADH enzyme powder or an immobilized enzyme prepared by 0.3 g of ADH and 10 mg of the GDH enzyme powder, were added; and stirring was performed for 20h at 35 °C. At the end of each round of the reaction, the immobilized enzyme was separated and reused in the next reaction. The number of reuse was checked. The conversion rate of the system was detected through HPLC. reaction data was shown in Table 8 below:

**Table 8**

| Enzyme | Resin name | Conversion rate | Number of cycles |
|---|---|---|---|
| ADH-Tb | Resin-free free enzyme | 98.7% | 1 |
| | LX-109s-IDA-Ni | >90% | 14 |
| | LX-1000EPN-IDA-Ni | >90% | 8 |
| | LX-EPHA-IDA-Ni | >90% | 11 |
| | LX-1000HA-IDA-Ni | >90% | 15 |
| | LX-109s | >90% | 5 |
| | LX-1000EPN | >90% | 4 |
| | LX-EPHA | >90% | 6 |
| | MIDA-Ni | >90% | 5 |
| | IMAC-Ni | >90% | 3 |
| ADH-Tb+GDH | Resin-free free enzyme | 98.6% | 1 |
| | LX-109s-IDA-Ni | >90% | 13 |
| | LX-1000EPN-IDA-Ni | >90% | 9 |
| | LX-EPHA-IDA-Ni | >90% | 11 |
| | LX-1000HA-IDA-Ni | >90% | 17 |
| | MIDA-Ni | >90% | 4 |
| | IMAC-Ni | >90% | 4 |

### Embodiment 8

Immobilization and application of an ene-reductase on affinity-modified amino- and epoxy-type methacrylic resins.

The difference between this embodiment and Embodiment 1 lied in that, the transaminase was equivalently replaced with the ene-reductase, and the cofactor was replaced with NAD(P)⁺ with equal mass.

Immobilized ene-reductase activity and reusability test:

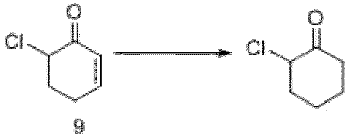

3 mL of a 0.1M phosphate buffer solution (pH7.0-8.0) was added to a 10 mL reaction bottle; then 100 mg of a substrate 9 was added; next, 10 mg of the NAD(P)+, 80 mg of ammonium formate, 2 mg of FDH, and 10 mg of an ERED free enzyme or an immobilized enzyme prepared by 10 mg of the ERED free enzyme or an immobilized enzyme prepared by 2 mg of the FDH and 10 mg of the ERED free enzyme were added. Reaction was performed for 16-20h at 30 °C. A conversion rate was tested. At the end of each round of the reaction, the immobilized enzyme was separated and reused in the next reaction. The number of reuse was checked. The conversion rate of the system was detected through gas chromatography. Reaction data was shown in Table 9 below:

**Table 9**

| Enzyme | Resin name | Conversion rate | Number of cycles |
|---|---|---|---|
| ERED-Chr | Resin-free free enzyme | 96.7% | 1 |
| | LX-109s-IDA-Ni | >90% | 12 |
| | LX-1000EPN-IDA-Ni | >90% | 6 |
| | LX-EPHA-IDA-Ni | >90% | 9 |
| | LX-1000HA-IDA-Ni | >90% | 11 |
| | LX-109s | >80% | 2 |
| | LX-1000EPN | >80% | 2 |
| | LX-EPHA | >80% | 2 |
| | MIDA-Ni | >90% | 3 |
| | IMAC-Ni | >90% | 2 |
| ERED-Sc | Resin-free free enzyme | 95.4% | 1 |
| | LX-109s-IDA-Ni | >90% | 10 |
| | LX-1000EPN-IDA-Ni | >90% | 8 |
| | LX-EPHA-IDA-Ni | >90% | 9 |
| | LX-1000HA-IDA-Ni | >90% | 14 |
| | LX-109s | >80% | 2 |
| | LX-1000EPN | >80% | 2 |
| | LX-EPHA | >80% | 2 |
| | MIDA-Ni | >90% | 2 |
| | IMAC-Ni | >90% | 2 |
| IRED-Str | Resin-free free enzyme | 96.3% | 1 |
| | LX-109s-IDA-Ni | >90% | 11 |
| | LX-1000EPN-IDA-Ni | >90% | 8 |
| | LX-EPHA-IDA-Ni | >90% | 10 |
| | LX-1000HA-IDA-Ni | >90% | 13 |
| | LX-109s | >80% | 2 |
| | LX-1000EPN | >80% | 2 |
| | LX-EPHA | >80% | 2 |
| | MIDA-Ni | >90% | 3 |
| | IMAC-Ni | >90% | 3 |
| IRED-Bc | Resin-free free enzyme | 99.4% | 1 |
| | LX-109s-IDA-Ni | >90% | 16 |
| | LX-1000EPN-IDA-Ni | >90% | 7 |
| | LX-EPHA-IDA-Ni | >90% | 15 |
| | LX-1000HA-IDA-Ni | >90% | 11 |
| | LX-109s | >80% | 3 |
| | LX-1000EPN | >80% | 2 |
| | LX-EPHA | >80% | 4 |
| | MIDA-Ni | >90% | 4 |
| | IMAC-Ni | >90% | 4 |
| IRED-Str+FDH | LX-1000HA-IDA-Ni | >90% | 15 |
| ERED-Sc+FDH | LX-1000HA-IDA-Ni | >90% | 16 |
| IRED-Str+FDH | LX-1000HA-IDA-Ni | >90% | 13 |
| IRED-Bc | LX-EPHA-IDA-Ni | >90% | 18 |

### Embodiment 9

Immobilization and application of an amino acid dehydrogenase on affinity-modified amino- and epoxy-type methacrylic resins.

The difference between this embodiment and Embodiment 1 lied in that, the transaminase was equivalently replaced with the amino acid dehydrogenase, and the cofactor was replaced with NAD⁺ with equal mass.

Immobilized amino acid dehydrogenase activity and reusability test:

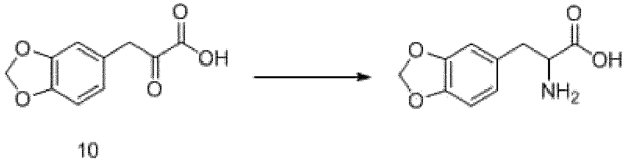

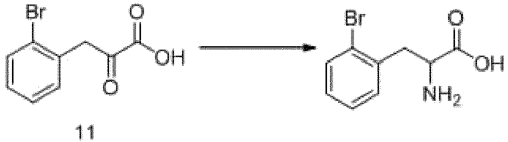

5 mL of a 0.1MTris-Cl buffer solution (pH8.0-9.0) was added to a 20 mL reaction bottle; 100 mg of a main raw material 10 or a main raw material 11 was dissolved, and 108 mg of ammonium chloride was added to regulate pH to 7.5-8.0; then 10-50 mg of the NAD⁺, 150 mg of glucose, 5 mg of GDH, and 10 mg of an AADH free enzyme or an immobilized enzyme prepared by 10 mg of the AADH free enzyme or a co-immobilized enzyme prepared by 5 mg of the GDH and 10 mg of the AADH free enzyme were added. Stirring was performed for 16-20h at 30 °C. A conversion rate was tested. At the end of each round of the reaction, the immobilized enzyme was separated and reused in the next reaction. The number of reuse was checked. The conversion rate of the system was detected through HPLC. reaction data was shown in Table 10 below:

**Table 10**

| Enzyme | Resin name | Raw material 10 | | Raw material 11 | |
|---|---|---|---|---|---|
| | | Conversion rate | Number of cycles | Conversion rate | Number of cycles |
| AADH-Bc | Resin-free free enzyme | 99.1% | 1 | 96.3% | 1 |
| | LX-109s-IDA-Ni | >90% | 15 | >90% | 13 |
| | LX-1000EPN-IDA-Ni | >90% | 12 | >90% | 9 |
| | LX-EPHA-IDA-Ni | >90% | 13 | >90% | 11 |
| | LX-1000HA-IDA-Ni | >90% | 14 | >90% | 12 |
| | LX-109s | >80% | 2 | >80% | 2 |
| | LX-1000EPN | >80% | 2 | >80% | 2 |
| | LX-EPHA | >80% | 2 | >80% | 1 |
| | MIDA-Ni | >90% | 3 | >90% | 3 |
| | IMAC-Ni | >90% | 6 | >90% | 3 |
| AADH-Bs | Resin-free free enzyme | 98.7% | 1 | 95.2% | 1 |
| | LX-109s-IDA-Ni | >90% | 13 | >90% | 13 |
| | LX-1000EPN-IDA-Ni | >90% | 11 | >90% | 14 |
| | LX-EPHA-IDA-Ni | >90% | 17 | >90% | 11 |
| | LX-1000HA-IDA-Ni | >90% | 14 | >90% | 15 |
| | LX-109s | >80% | 2 | >80% | 2 |
| | LX-1000EPN | >80% | 2 | >80% | 2 |
| | LX-EPHA | >80% | 2 | >80% | 2 |
| | MIDA-Ni | >90% | 3 | >90% | 2 |
| | IMAC-Ni | >90% | 2 | >90% | 2 |
| AADH-Ti | Resin-free free enzyme | 98.8% | 1 | 96.1% | 1 |
| | LX-109s-IDA-Ni | >90% | 13 | >90% | 13 |
| | LX-1000EPN-IDA-Ni | >90% | 9 | >90% | 10 |
| | LX-EPHA-IDA-Ni | >90% | 16 | >90% | 17 |
| | LX-1000HA-IDA-Ni | >90% | 17 | >90% | 15 |
| | LX-109s | >80% | 2 | >80% | 2 |
| | LX-1000EPN | >80% | 2 | >80% | 2 |
| | LX-EPHA | >80% | 2 | >80% | 2 |
| | MIDA-Ni | >90% | 2 | >90% | 2 |
| | IMAC-Ni | >90% | 2 | >90% | 2 |
| AADH-Ti-1 | Resin-free free enzyme | 99.8% | 1 | 96.9% | 1 |
| | LX-109s-IDA-Ni | >90% | 16 | >90% | 12 |
| | LX-1000EPN-IDA-Ni | >90% | 11 | >90% | 9 |
| | LX-EPHA-IDA-Ni | >90% | 18 | >90% | 15 |
| | LX-1000HA-IDA-Ni | >90% | 17 | >90% | 13 |
| | LX-109s | >80% | 3 | >80% | 3 |
| | LX-1000EPN | >80% | 2 | >80% | 2 |
| | LX-EPHA | >80% | 4 | >80% | 3 |
| | MIDA-Ni | >90% | 3 | >90% | 3 |
| | IMAC-Ni | >90% | 4 | >90% | 2 |
| AADH-Ti-2 | Resin-free free enzyme | 99.6% | 1 | 97.0% | 1 |
| | LX-109s-IDA-Ni | >90% | 15 | >90% | 14 |
| | LX-1000EPN-IDA-Ni | >90% | 13 | >90% | 16 |
| | LX-EPHA-IDA-Ni | >90% | 19 | >90% | 19 |
| | LX-1000HA-IDA-Ni | >90% | 21 | >90% | 18 |
| | LX-109s | >80% | 3 | >80% | 3 |
| | LX-1000EPN | >80% | 4 | >80% | 4 |
| | LX-EPHA | >80% | 2 | >80% | 2 |
| | MIDA-Ni | >90% | 4 | >90% | 4 |
| | IMAC-Ni | >90% | 3 | >90% | 3 |
| AADH-TL | Resin-free free enzyme | 98.7% | 1 | 96.7% | 1 |
| | LX-109s-IDA-Ni | >90% | 9 | >90% | 12 |
| | LX-1000EPN-IDA-Ni | >90% | 11 | >90% | 11 |
| | LX-EPHA-IDA-Ni | >90% | 13 | >90% | 12 |
| | LX-1000HA-IDA-Ni | >90% | 8 | >90% | 13 |
| | LX-109s | >80% | 2 | >80% | 2 |
| | LX-1000EPN | >80% | 2 | >80% | 2 |
| | LX-EPHA | >80% | 2 | >80% | 2 |
| | MIDA-Ni | >90% | 3 | >90% | 2 |
| AADH-TL-1 | Resin-free free enzyme | 98.9% | 1 | 97.1% | 1 |
| | LX-109s-IDA-Ni | >90% | 9 | >90% | 11 |
| | LX-1000EPN-IDA-Ni | >90% | 10 | >90% | 9 |
| | LX-EPHA-IDA-Ni | >90% | 11 | >90% | 10 |
| | LX-1000HA-IDA-Ni | >90% | 6 | >90% | 9 |
| | LX-109s | >80% | 2 | >80% | 2 |
| | LX-1000EPN | >80% | 2 | >80% | 2 |
| | LX-EPHA | >80% | 2 | >80% | 2 |
| | MIDA-Ni | >90% | 4 | >90% | 4 |
| AADH-TL-2 | Resin-free free enzyme | 99.1% | 1 | 97.1% | 1 |
| | LX-109s-IDA-Ni | >90% | 14 | >90% | 15 |
| | LX-1000EPN-IDA-Ni | >90% | 11 | >90% | 13 |
| | LX-EPHA-IDA-Ni | >90% | 10 | >90% | 10 |
| | LX-1000HA-IDA-Ni | >90% | 11 | >90% | 15 |
| | LX-109s | >80% | 2 | >80% | 2 |
| | LX-1000EPN | >80% | 2 | >80% | 2 |
| | LX-EPHA | >80% | 1 | >80% | 1 |
| | MIDA-Ni | >90% | 4 | >90% | 4 |
| AADH-Bc+GDH | LX-109s-IDA-Ni | >90% | 15 | >90% | 13 |
| AADH-Bs+GDH | LX-EPHA-IDA-Ni | >90% | 16 | >90% | 12 |
| AADH-Ti+GDH | LX-EPHA-IDA-Ni | >90% | 12 | >90% | 12 |
| | LX-1000HA-IDA-Ni | >90% | 19 | >90% | 17 |
| AADH-Ti-1+GDH | LX-1000HA-IDA-Ni | >90% | 19 | >90% | 12 |

### Embodiment 10

Immobilization and application of a transaminase on amino- and epoxy-type methacrylic resins after affinity modification with different metal ions. The difference between this embodiment and Embodiment 1 lied in that, equimolar nickel ions were respectively replaced with cobalt ions and copper ions.

Immobilized transaminase activity and reusability test:

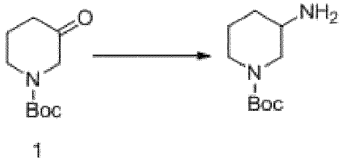

0.1 g of a main raw material 1 was added to a 10 mL reaction bottle; 4eq isopropylamine hydrochloride and 1 mg of the PLP were added; the 0.1M phosphate buffer solution 7.0 was replenished to make the final volume of the reaction solution up to 1 mL; then 0.01 g of the enzyme powder or the immobilized enzyme, which was prepared by 0.01 g of the enzyme powder, was added; and stirring was performed for 16-20h at 30 °C. A conversion rate was tested. At the end of each round of the reaction, the immobilized enzyme was separated and reused in the next reaction. The number of reuse was checked. The conversion rate of the system was detected through HPLC. reaction data was shown in Table 11 below:

**Table 11**

| Enzyme | Resin name | Conversion rate | Number of cycles |
|---|---|---|---|
| TA-Cv | Resin-free free enzyme | >98% | 1 |
| | LX-1000HA-IDA-Ni | >90% | 15 |
| | LX109s-IDA-Ni | >90% | 12 |
| | LX-1000HA-IDA-Co | >90% | 10 |
| | LX109s-IDA-Co | >90% | 9 |
| | LX-1000HA-IDA-Cu | >90% | 9 |
| | LX109s-IDA-Cu | >90% | 11 |

The above are only the preferred embodiments of the present disclosure and are not intended to limit the present disclosure. For those skilled in the art, the present disclosure may have various modifications and variations. Any modifications, equivalent replacements, improvements, and the like made within the spirit and principle of the present disclosure all fall within the scope of protection of the present disclosure.

## Claims

1. An enzyme immobilization carrier, **characterized in that** the enzyme immobilization carrier comprises a resin matrix, a -N(CH₂COOH)₂ group linked on the resin matrix by means of a chemical bond, and a metal ion adsorbed on the -N(CH₂COOH)₂ group in a chelating manner through coordination, wherein, the resin matrix is an amino-type methacrylic resin or an epoxy-type methacrylic resin, and the resin matrix has a particle size of 200-700 µm.

2. The enzyme immobilization carrier according to claim 1, **characterized in that** in the amino-type methacrylic resin, the amino-type methacrylic resin has an amino functional group with a C2 or C6 length carbon chain arm, and the content of the amino is 30-80 µmol/g;
preferably, Seplite^{®}LX-1000HA, Seplite^{®}LX-1000EPN, Seplite^{®}LX-EPHA, Seplite^{®}LX-1000EA, Lifetech^{™}ECR8309, Lifetech^{™}ECR8409,ESR-1, ESR-3, or ESQ-1; more preferably, the amino-type methacrylic resin is one or more of Seplite^{®}LX-1000HA, Seplite^{®}LX-1000EPN, Seplite^{®}LX-EPHA, or Lifetech^{™}ECR8309;
preferably, the epoxy-type methacrylic resin has an epoxide equivalent of 2-5 µmol/g;
preferably, the epoxy-type methacrylic resin is one or more of Seplite^{®}LX-1000EP, Seplite^{®} LX-103B, EP200, Seplite^{®}LX-107B, Seplite^{®}LX-1000SW, Seplite^{®}LX-1000SD, Seplite^{®} LX-109s, Seplite^{®}LX-1000HFA, Lifetech^{™}ECR8285, Lifetech^{™}ECR8204, Lifetech^{™}ECR8209, ES-1, ES103, ES-101, ReliZyme^{™}HFA403, or ReliZyme^{™}EC-HFA; more preferably, the epoxy-type methacrylic resin is one or more of Seplite^{®}LX-1000EP, Seplite^{®}LX-109s, Seplite^{®} LX-1000HFA, or ReliZyme^{™}EC-HFA;
preferably, the metal ion is a nickel ion, an iron ion, a copper ion or a cobalt ion.

3. An immobilized enzyme, **characterized in that** the immobilized enzyme comprises the enzyme immobilization carrier of claim 1 or 2 and an enzyme immobilized thereon.

4. The immobilized enzyme according to claim 3, **characterized in that** the enzyme is selected from one or more of a transaminase, a ketoreductase, an alcohol dehydrogenase, a formate dehydrogenase, a glucose dehydrogenase, a monooxygenase, an ene-reductase, an imine reductase, and an amino acid dehydrogenase; wherein,
the transaminase is a transaminase derived from *Chromobacterium violaceum DSM30191,* or a transaminase derived from *Arthrobactercitreus,* or a transaminase derived from *Actinobacteria,* or a transaminase derived from *Sciscionella sp. SE31;*
the ketoreductase is a carbonyl reductase derived from *Acetobacter sp. CCTCC M209061,* or a ketoreductase derived from *Sporobolomyces salmonicolor,*
the alcohol dehydrogenase is derived from *Thermoanaerobium brockii;*
the formate dehydrogenase is a formate dehydrogenase derived from *Candida boidinii;*
the glucose dehydrogenase is a glucose dehydrogenase derived from *Lysinibacillus sphaericus G10;*
the monooxygenase is a cyclohexanone monooxygenase derived from *Rhodococcus sp. Phil,* or a cyclohexanone monooxygenase derived from *Rhodococcus ruber-SD1,* or a cyclohexanone monooxygenase derived from *Brachymonas petroleovorans;*
the ene-reductase is an enoyl reductase derived from *Saccharomyces cerevisiae,* or an ene-reductase derived from *Chryseobacterium sp. CA49;*
the imine reductase is an imine reductase derived from *Streptomyces sp.,* or an imine reductase derived from *Bacillus cereus;*
the amino acid dehydrogenase is a leucine dehydrogenase derived from *Bacillus cereus,* or a phenylalanine dehydrogenase derived from *Bacillus sphaericus;* or an amino acid dehydrogenase derived from *Thermoactinomyces intermedius ATCC33205,* or an amino acid dehydrogenase derived from *Thermosyntropha lipolytica.*

5. A method for preparing the enzyme immobilization carrier of claim 1 or 2, **characterized by** comprising the following steps:
providing a resin matrix;
linking an -N(CH₂COOH)₂ group onto the resin matrix by means of a chemical bond;
absorbing a metal ion onto the -N(CH₂COOH)₂ group in a chelating manner through coordination to obtain the enzyme immobilization carrier;
wherein, the resin matrix is the amino-type methacrylic resin or the epoxy-type methacrylic resin, and the resin matrix has a particle size of 200-700 µm.

6. The method for preparing the enzyme immobilization carrier according to claim 5, **characterized in that** when the resin matrix is the amino-type methacrylic resin, the amino-type methacrylic resin is mixed with sodium chloroacetate for nucleophilic substitution, such that the -N(CH₂COOH)₂ group is linked onto the amino-type methacrylic resin.

7. The method for preparing the enzyme immobilization carrier according to claim 6, **characterized in that** after an aqueous solution of the amino-type methacrylic resin and the sodium chloroacetate is stirred at 20-25 °C for 30-60 min, the reaction system is regulated to a pH of 9-10 with 1-2 mol/L of an aqueous alkaline solution, and then heated up to 70-80 °C for reaction for 20-30 h at a N₂ atmosphere, such that the -N(CH₂COOH)₂ group is linked onto the amino-type methacrylic resin;
preferably, the aqueous alkali solution is a sodium carbonate solution, a sodium hydroxide solution or a lithium hydroxide solution.

8. The method for preparing the enzyme immobilization carrier according to claim 5, **characterized in that** when the resin matrix is the epoxy-type methacrylic resin, the epoxy-type methacrylic resin is mixed with Iminodiacetic acid disodium for addition reaction, such that the -N(CH₂COOH)₂ group is linked onto the epoxy-type methacrylic resin.

9. The method for preparing the enzyme immobilization carrier according to claim 8, **characterized in that** after an aqueous solution of the epoxy-type methacrylic resin and the Iminodiacetic acid disodium is stirred at 20-25 °C for 30-60 min, the reaction system is heated up to 60-70 °C for reaction for 18-24 h at a N₂ atmosphere, such that the -N(CH₂COOH)₂ group is linked onto the epoxy-type methacrylic resin.

10. The method for preparing the enzyme immobilization carrier according to any one of claims 5 to 9, **characterized in that** after the step of linking the -N(CH₂COOH)₂ group onto the resin matrix by means a chemical bond, a metal salt solution is added to the reaction system for complex reaction, such that the metal ion is absorbed on the -N(CH₂COOH)₂ group by coordination, thus obtaining the enzyme immobilization carrier;
preferably, in the complex reaction, a reaction temperature is 20-30 °C and a reaction time is 1-4 h;
preferably, the metal salt solution is an aqueous solution of nickel chloride, an aqueous solution of copper sulfate, an aqueous solution of ferrous chloride or an aqueous solution of cobalt chloride;
preferably, in the metal salt solution, a mass ratio of the metal ion to the resin ball matrix is (0.05-0.1):1.

11. The method for preparing the enzyme immobilization carrier according to claim 6, **characterized in that** a mass ratio of the sodium chloroacetate to the amino-type methacrylic resin is (5-10):1.

12. The method for preparing the enzyme immobilization carrier according to claim 8, **characterized in that** a mass ratio of the Iminodiacetic acid disodium to the epoxy-type methacrylic resin ball is (5-10):1.

13. A method for preparing the immobilized enzyme of claim 3 or 4, **characterized by** comprising the following steps: performing an immobilization reaction on the enzyme immobilization carrier of claim 1 or 2 and an enzyme to obtain the immobilized enzyme.

14. The method for preparing the immobilized enzyme according to claim 13, **characterized in that** the method for preparing the immobilized enzyme comprises the following steps:
dispersing the enzyme immobilization carrier into a first solvent to form a dispersion liquid, wherein the first solvent is a mixed solution of a phosphate buffer solution, a sodium chloride aqueous solution and an imidazole buffer solution;
reacting the dispersion liquid with an enzyme solution containing the enzyme, such that the enzyme and the enzyme immobilization carrier are subjected to immobilization reaction to obtain the immobilized enzyme.

15. The method for preparing the immobilized enzyme according to claim 14, **characterized in that** in the first solvent, the phosphate buffer solution has a concentration of 0.1-0.2 mol/L; the sodium chloride aqueous solution has a concentration of 0.5-1 mol/L; and the imidazole buffer solution has a concentration of 0.05-0.1 mol/L.

16. The method for preparing the immobilized enzyme according to claim 14, **characterized in that** in the reaction process of the dispersion liquid and the enzyme solution, a reaction temperature is 20-25 °C, and a reaction time is 16-24 h; preferably, per gram of the enzyme immobilization carrier is reacted with 4-8 mL of the enzyme solution, and the enzyme solution has a protein content of 20-25 mg/mL.
